# EUROPEAN PATENT APPLICATION

(11) **EP 4 316 390 A1**
(43) Date of publication of application: **07.02.2024**
(21) Application number: 22199316.5
(22) Date of filing: 01.10.2022
(51) Int. Cl.: A61B 17/128, A61B 17/00

(54) **SURGICAL CLIP APPLIER WITH LOCKING STRUCTURE**

(30) Priority: 04.08.2022 TW 111129358
(71) Applicant: Taiwan Surgical Corporation, Hsinchu County 302 (TW)
(72) Inventor: TSENG, HUNG-YIN, 302 Zhubei City, Hsinchu County (TW); HSIAO, WEN CHEN, 302 Zhubei City, Hsinchu County (TW)
(74) Representative: Cabinet Chaillot

(57) **Abstract**

A surgical clip applier has an outer tube (10), wherein the outer tube (10) has a shifting plate and a fixing plate at the bottom side of the shifting plate. The shifting plate and the fixing plate form a clip storage space therebetween. A clip pusher (16) slides along the clip storage space by the movement of the shifting plate. After the last clip is discharged, the shifting plate pushes the clip pusher (16) into a jaw (14) which is mounded at the distal end of the outer tube (10). The jaw (14) is prevented from closing and the problem of damaging a blood vessel can be avoided.

## Description

### FIELD OF INVENTION

The present invention relates to a clip applier, especially to a clip applier with a locking structure.

### BACKGROUND OF THE INVENTION

In a surgical operation process, in order to avoid blood over losing from a surgical operation site, a surgical clip applier is usually applied to close a blood vessel by applying a clip on the blood vessel. A conventional way of using the surgical clip applier is locating the jaw which is mounted at a distal end of surgical clip applier to a position near the target blood vessel by inserting a long rod-shaped surgical clip applicator into the surgical operation site of the human body. Then, a handle at a proximal end of surgical clip applier is operated to make the jaw apply a clip onto the blood vessel.

However, though the arranged clips can be delivered to the jaw sequentially, it cannot be clearly noticed that whether the last clip is run out when using the clip applier. With the careless attention, it is easy for the jaw to directly clamp the blood vessel and damage the vascular tissue by operate the surgical clip applier continuously after the last clip is discharged. It not only affects the operation process, but also causes unnecessary harm to the patient's body

Therefore, it is an urgent program in the related field to develop a surgical clip applier with a locking function after the last clip is discharged.

### SUMMARY OF THE INVENTION

1. To overcome the aforementioned shortcomings of the prior surgical clip applier, the present invention provides a surgical clip applier with a locking structure comprises an outer tube with a jaw protruding from a distal end of the outer tube and a handle mounted at a proximal end of the outer tube, in the outer tube from top to bottom sequentially arranged comprising:
a shifting plate with a proximal end connected to the handle presenting a reciprocate movement relative to the outer tube from a movement of the handle, multiple shifting elastic leaves arranged apart to each other in a predetermined distance on the shifting plate along the axis, each shifting elastic leaf protruded toward the distal end downwardly, and a feeding portion mounted at the distal end of the shifting plate moving the distal most clip into the jaw; and
a fixing plate, multiple fixing elastic leaves arranged apart to each other on the fixing plate corresponding to the shifting elastic leaves, each fixing elastic leaf protrudes toward the distal end upwardly, a storage space defined at the space between the shifting plate and the fixing plate, the storage space holding multiple clips in sequence inside, a clip pusher moved along the storage space by the movement of shifting plate and pushing the proximal most clip toward to the distal end of outer tube; wherein
after the last clip is ejected, the clip pusher is moved to a distal most position, when the handle is triggered again, the feeding portion push the clip pusher into the jaw.

The present invention forms the locking structure when the handle is triggered again that the feeding portion may push the clip pusher into the clamping space. With the revealed features, the surgical clip applier with the locking structure cannot be trigger further that overcome the shortage of the prior surgical clip applier which cannot be clearly noticed that the last clip is run out. Combining the design of the effect of preventing the reverse movement with the tooth-locking structure, the pressing handle can be locked and cannot be worked by any other pression again. Specifically achieves an advantage that using a tactile-cue to notice the user that the clip in the surgical clip applier is run out, and prevents the misused of the surgical clip applier when preparing or using.

### BRIEF DESCRIPTION OF THE DRAWINGS

Fig. 1 is a perspective view of a preferred embodiment of a surgical clip applier in accordance with the present invention;
Fig. 2A is an enlarged cross sectional operational side view of a first operation of the surgical clip applier in Fig. 1;
Fig. 2B is an enlarged cross sectional operstional side view of a second operation of the surgical clip applier in Fig, 1;
Fig. 2C is an enlarged cross sectional operational side view of a third operation of the surgical clip applier in Fig. 1;
Fig. 3 is an enlarged partially exploded perspective view of the surgical clip applier in Fig. 1;
Fig. 4 is an enlarged cross sectional side view of the surgical clip applier in in Fig. 3;
Fig. 5 is an enlarged perspective view of a jaw of the surgical clip applier in Fig. 1; and
Fig. 6 is a side view of a handle of the surgical clip applier in Fig, 1.

### DETAILED DESCRIPTION OF THE PREFERRED EMBODIMENTS

In order to make purposes, technical solutions, and advantages of the present invention to be clearer, the following content provides some preferred embodiments in accordance with the present invention.

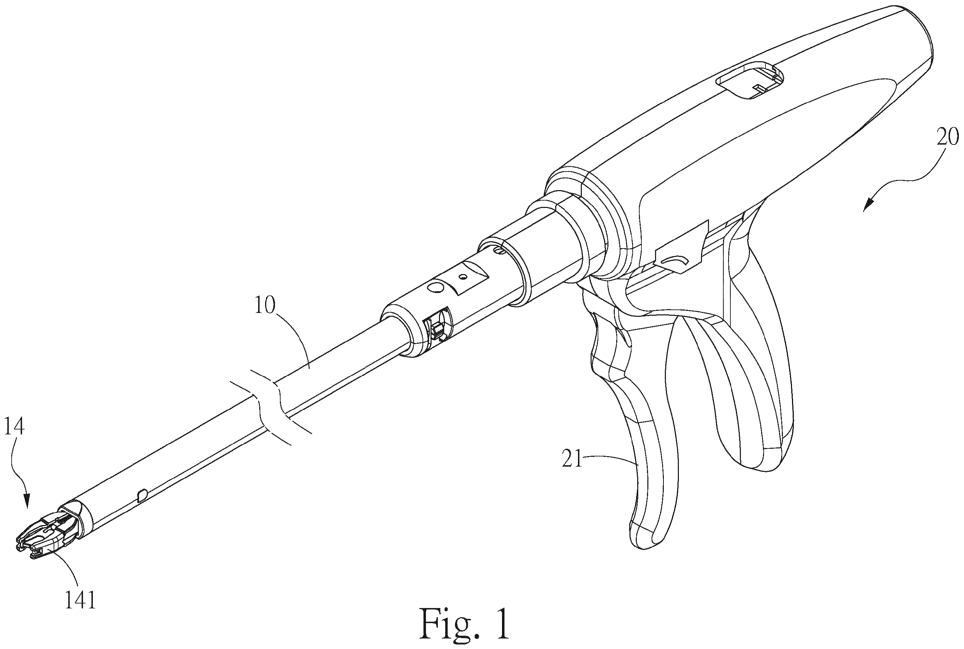

With reference to Fig. 1 to Fig. 3, a surgical clip applier in accordance with the present invention comprises a rod-shaped outer tube 10 extending along an axis, and a handle 20 mounted at a proximal end of the outer tube 10. A jaw 14 protrudes from the distal end of the outer tube 10 and has two jaw units 141 which are spaced apart from each other and are mounted respectively on opposite sides of the jaw 14. The space between two jaw units 141 defines a clamping space which can be narrowed or expanded according to the close or open movement from the two jaw units 141 driven by handle 20.

A shifting plate 11 and a fixing plate 12 are mounted in the outer tube 10 and are spaced apart from each other with the fixing plate 12 is located below the shifting plate 11. A storage space 13 is defined at the space between the shifting plate 11 and the fixing plate 12 to hold multiple clips A in sequence inside. The proximal end of the shifting plate 11 is connected to the handle 20, and the handle 20 drives the shifting plate 11 to present a reciprocating movement with a predetermined distance.

Multiple shifting elastic leaves 111 are arranged at spaced intervals on the shifting plate 11 along the axis. Each shifting elastic leaf 111 protrudes toward the distal end downwardly, and is arranged apart to each other with the predetermined distance preferably. The distal end of the shifting plate 11 forms a feeding portion 15 which is applied to push the proximal most clip A to the clamping space.

The fixing plate 12 is fixed in the outer tube 10. Multiple fixing elastic leaves 121, corresponding to the shifting elastic leaves 111 at positions, are arranged at spaced intervals on the fixing plate 12 along the axis. Each fixing elastic leaf 121 protrudes toward the distal end upwardly, and is arranged apart to each other with the predetermined distance. Each shifting elastic leaf 111 and a corresponding one of the fixing elastic leaves 121 have a corresponding position and protrude toward each other, such that the storage space 13 can be divided into multiple clip storage units C.

A block-like clip pusher 16 is located at the proximal side of the proximal most clip A and can be moved along the storage space 13. The clip pusher 16 can push the proximal most clip A and generates a pushing force to urge the multiple clips A to move toward the distal end of the outer tube 10. When the feeding portion 15 pushes the distal most clip A to the clamping space, the multiple remaining clips A can be pushed toward and supplemented to the distal end of the outer tube 10 with the pushing force.

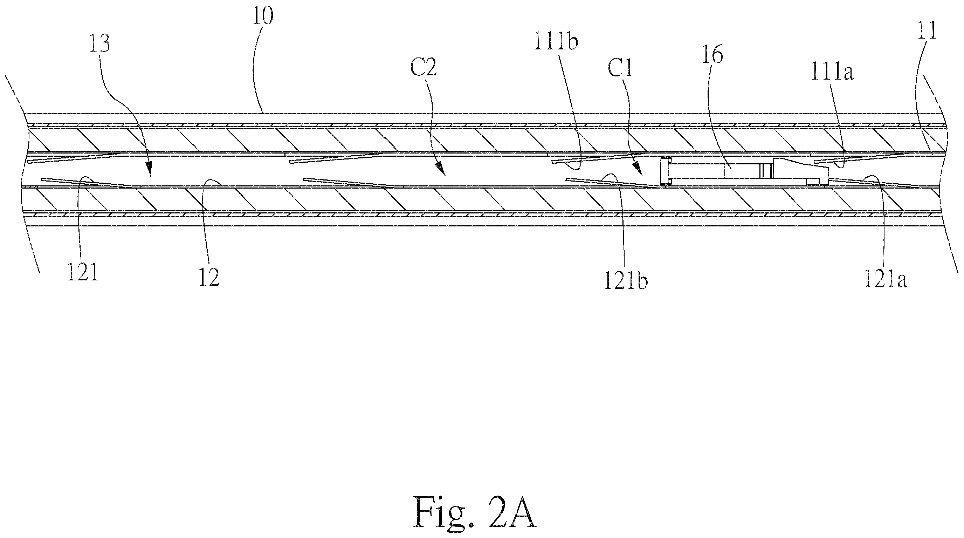

Referring to Fig.2A to Fig.2C, an operation embodiment of the present invention to push the clips is provided. In Fig. 2A, since an initiated position, the clip pusher 16 is placed at a first clip storage unit C1. A first shifting elastic leaf 111a and a first fixing elastic leaf 121a, with up-down setting, abut with the proximal side of the clip pusher 16 to prevent the clip pusher 16 from moving toward the proximal end of the outer tube 10.

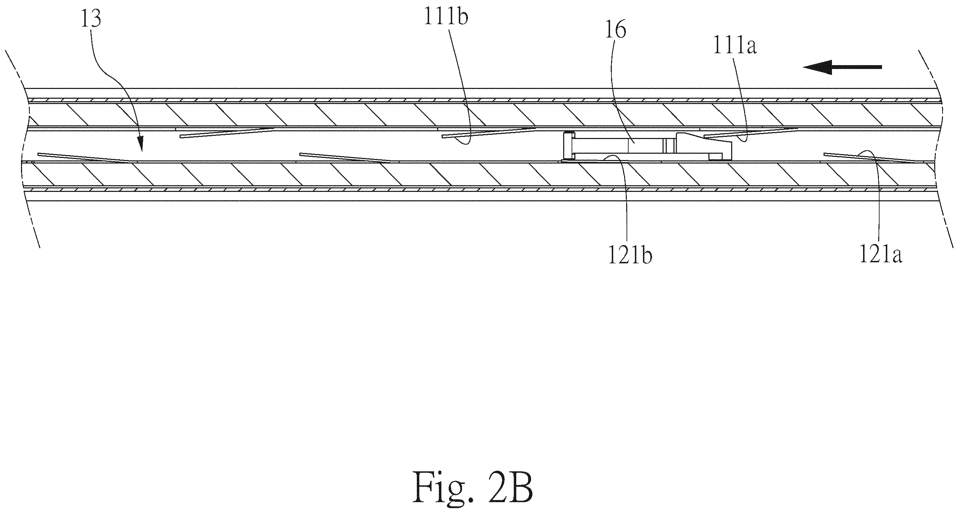

In Fig. 2B, when the handle 20 is triggered, the shifting plate 11 is slid toward the distal end of the outer tube 10 by the movement of the handle 20. At the meantime, the feeding portion 15 which is mounted at the distal end of the shifting plate 11 pushes the proximal most clip A to the clamping space. With the distal movement of the shifting plate 11, the first shifting elastic leaf 111a drives the clip pusher 16 to move distally to a second clip storage unit C2. When the shifting plate 11 brings the clip pusher 16 passing over a second fixing elastic leaf 121b at the front end below. The second fixing elastic leaf 121b is deformed toward the fixing plate 12 elastically by urging from the clip pusher 16. Then the clip pusher 16 passes over the second fixing elastic leaf 121b which is deformed, and can be shifted to the second clip storage unit C2.

After the shifting plate 11 brings the clip pusher 16 to pass through the second fixing elastic leaf 121b and makes the clip pusher 16 shift to the second clip storage unit C2, the second fixing elastic leaf 121b returns to the original position which protrudes toward the distal end upwardly by its own elastic resilience feature. The second fixing elastic leaf 121b abuts the proximal side of the clip pusher 16 to prevent the clip pusher 16 from moving toward the proximal end of the outer tube 10. At the meantime, the jaw mounted at the distal end of the outer tube 10 is closed, and urges the clip A which is placed at the clamping space to be deformed.

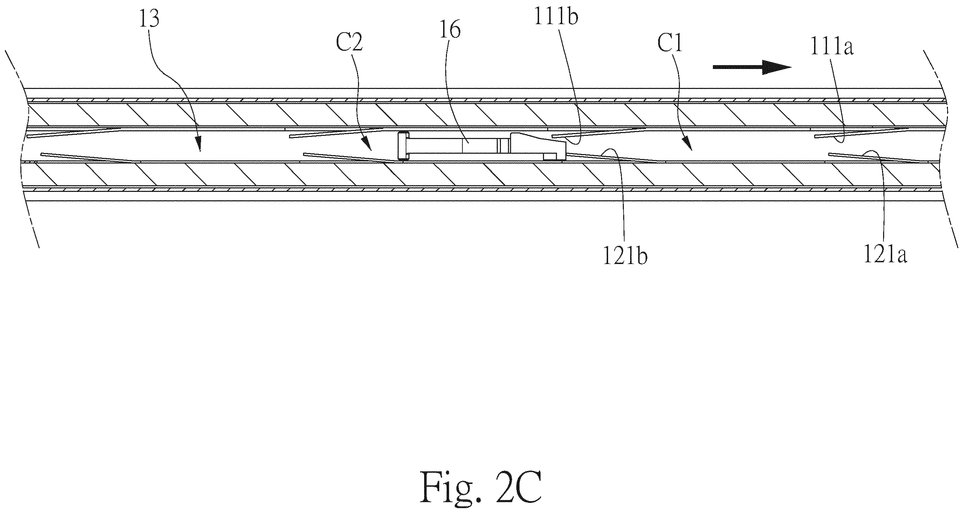

Referring to the Fig.2C, when the handle 20 is released, the shifting plate 11 moves toward the proximal end, and the first shifting elastic leaf 111a is separated from the proximal side of the clip pusher 16. When the shifting plate 11 brings a second shifting elastic leaf 111b to pass over the clip pusher 16, the second shifting elastic leaf 111b is deformed toward the shifting plate 11 elastically by urging from the clip pusher 16. Then the second shifting elastic leaf 111b can pass over the clip pusher 16 and the second shifting elastic leaf 111b can align and abut with the proximal side of the clip pusher 16. With features mentioned before, the clip pusher 16 can be pushed toward the distal end of the outer tube 20 by triggering the handle 20 each time, the multiple clips A which are arranged sequenced in the storage space 13 also can be drives to move forwardly for supplement.

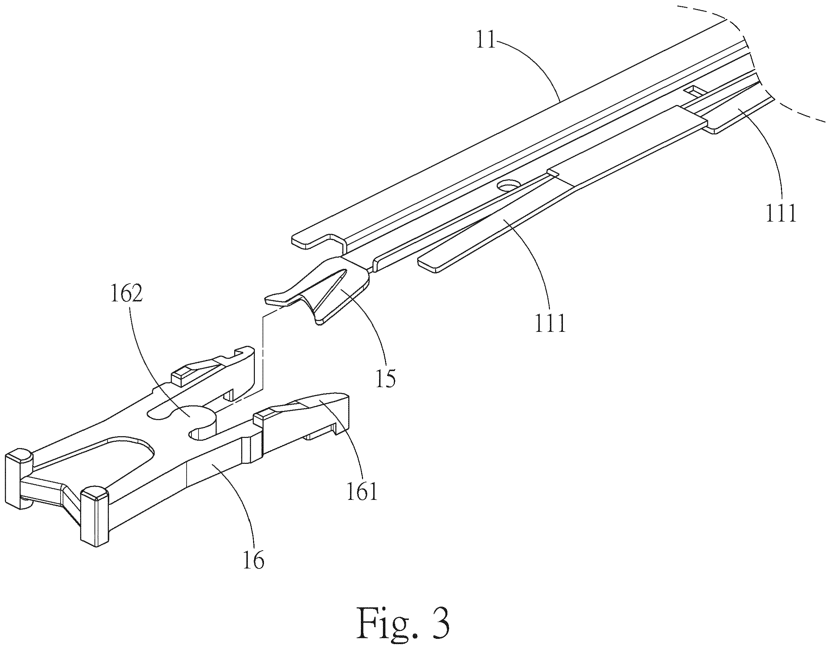

Please then referring to the Fig.3, one side (as a right side in this embodiment) of the shifting plate 11 has the multiple shifting elastic leaves 111 protruding toward the distal end downwardly. Another side (as a middle side in this embodiment) of shifting plate 11 forms the feeding portion 15. The staggered arrangement of the multiple shifting elastic leaves 111 and the feeding portion 15 can avoid the problem of structural interference when the shifting plate 11 pushing the frontmost clip A.

The proximal side of the clip pusher 16 has a pushing portion 161 which is located at a position corresponding to the multiple shifting elastic leaves 111, and has an abutting portion 162 with a position corresponding to the center of the feeding portion 15. Preferably, the abutting portion 162 is located at the distal side of the pushing portion 161 in accordance with the relative position of the feeding portion 15 and the frontmost shifting elastic leaf 111. More preferably, the pushing portion 161 has a shape which can match an edge contour of the clip A or the distal end of the feeding portion 15, such that the feeding portion 15 can exhibit a stable and smooth pushing effect when pushing the pushing portion 161.

When the shifting plate 11 is slid toward the distal end of the outer tube 10 by the movement of the handle 20. The shifting elastic leaves 111 can detachably combine with the pushing portion 161, and the shifting plate 11 pushes the clip pusher 16 to avoid the problem of the displacement or deformation of the shifting elastic leaves 111 when pushing. The pushing portion 161 can be a notch which is defined in the proximal side surface of the clip pusher 16. The shifting elastic leaves 111 can be fixed by being inserted into the notch when moving distally.

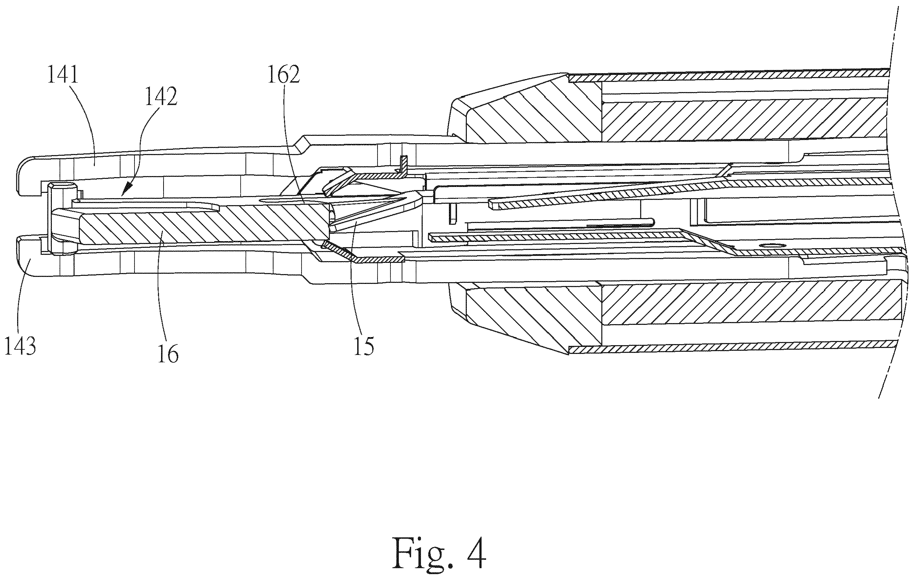

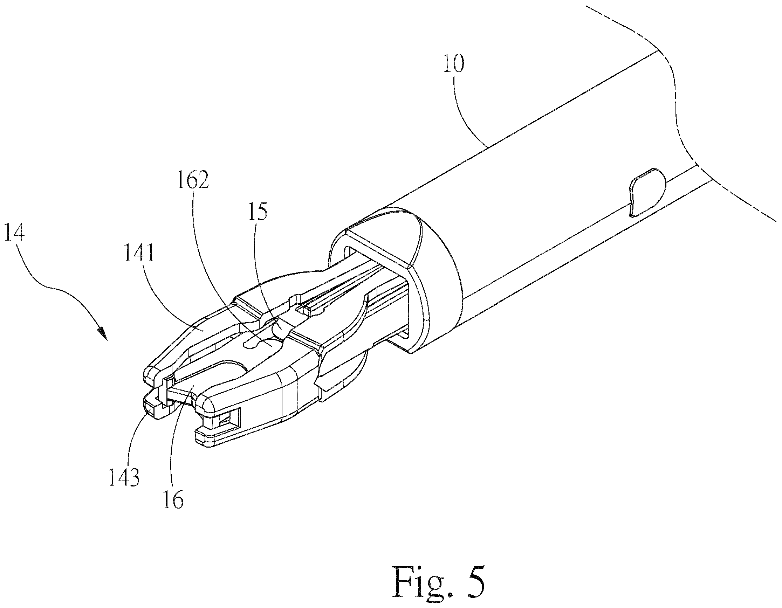

Referring to the Fig.3 to Fig.5, when the last clip A is placed into the clamping space, the clip pusher 16 is moved to a distal most position by the movement of the shifting plate 11 simultaneously. At the same time, the feeding portion 15 abuts the proximal side of the abutting portion 162. When the handle 20 is triggered again, the shifting plate 11 drives the feeding portion 15 to push the clip pusher 16 into the clamping space. The jaw 14 cannot compress the clip pusher 16 to deform, so that the jaw 14 and the clip pusher 16 are engaged with each other. With the engaged relationship of the jaw 14 and the clip pusher 16, the clip applier generates a locking structure and overcomes the problem of that the jaw usually clamps the blood vessel and damages the vascular tissue by operate the surgical clip applier continuously after the last clip is discharged.

Moreover, the jaw 14 has two jaw units 141 which are spaced apart from each other and are mounted respectively on opposite sides of the jaw 14. The space between two jaw units 141 defines a clamping space which can be narrowed or expanded according to the close or open movement from the two jaw units 141 driven by handle 20. One side of the two jaw units 141 corresponding to each other can be defined as an inner wall. Each inner wall is provided with a feeding notch 142 which extends along the axis. The two feeding notches 142 correspond to each other in position. Therefore, two side of the clip A or the clip pusher 16 can follow the feeding notches 142 to move into the clamping space to prevent the clip A or the clip pusher 16 from falling off when moving to the clamping space from the outer tube 10. The distal end of the two jaw units 141 extends a stop portion 143 radially in order to axially stop the clip A or the clip pusher 16 falling off from the two jaw units 141 when moving. With the features of the jaw 14 mentioned before, the jaw 14 can express the excellent holding feature and make sure the locking structure is definitely be generated.

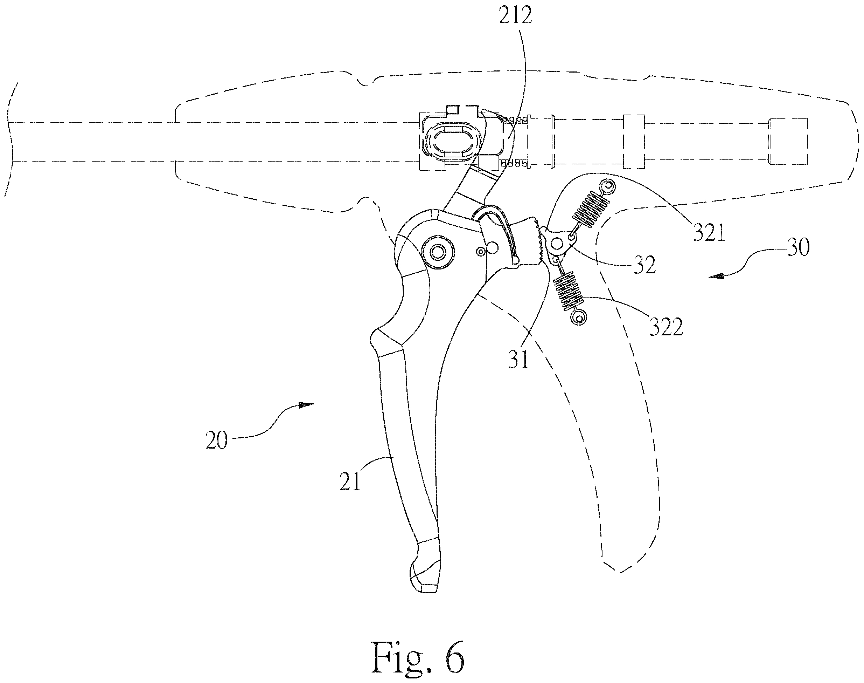

With reference to Fig.6, the feature of the handle 20 is not limited in the present invention. The present embodiment applies the features disclosed in the previous documents TWI703019. The handle 20 is a trigger structure including a fixing handle and a pressing handle 21. The pressing handle 21 is a rod-like shape and forms a pivot portion at the top side of the pressing handle 21. When the bottom side of the pressing handle 21 is pivoted relative to the fixing handle, the shifting plate 11 may be moved toward the distal end of outer tube 10.

A hook section 212extends upwardly from the pressing handle 21 . When the pressing handle 21 is pivoted toward the fixing handle, the hook section 212 may drive the feeding rod 11 to move toward the distal end of outer tube 10 to make the clip A be fired when the handle 20 is triggered.

A tooth-locking structure 30 is located near the pressing handle 21 and comprises a ratchet 31 and a toothed block 32. The toothed block 32 forms a toothed edge 321 facing the ratchet 31. When the handle 20 is triggered or released, the toothed edge 321 engages and moves along the ratchet 31 unidirectionally according to a pivot direction of the pressing handle 21. The toothed block 32 is pivoted toward the pivot direction at the same time. Two connect springs 322, at the other side of the toothed edge 321, are respectively settled at two opposite sides of the toothed block 32. After the toothed edge 321 moves unidirectionally along the ratchet 31, one of the connect springs 322 can provide a reverse pulling force to make the toothed block 32 move toward to a counter direction. The reverse pulling force generated by the two connect springs 322 can smooth the engagement of the toothed edge 321 and the ratchet 31 when the pressing handle 21 is pivoted for being triggered or released.

When the handle 20 is triggered again after the last clip A is ejected, since the engagement of the jaw14 and the clip pusher 16, the jaw 14 cannot be closed and the handle 20 cannot be pressed further. The toothed edge 321 is stuck in at least a part of the ratchet 31 and cannot be pivoted, so that the effect of preventing the reverse movement of the handle 20 is achieved.

After the last clip A is ejected and the clip pusher 16 is moved, by the shifting plate 11, to a distal most position where the proximal end of clip pusher 16 is corresponding to the distal end of the feeding portion 15. The clip applier in accordance with the present invention then forms the locking structure when the handle 20 is triggered again and the feeding portion 15 may push the clip pusher 16 into the clamping space. With the revealed features, the surgical clip applier with the locking structure cannot be triggered further to overcome the shortage of the prior surgical clip applier which cannot be clearly noticed that the last clip is run out.

Combining the design of the effect of preventing the reverse movement with the tooth-locking structure 30, the pressing handle 21 can be locked and cannot be worked by the any other pression again. Specifically achieves an advantage that using a tactile-cue to notice the user that the clip A in the surgical clip applier is run out, and prevents the misused of the surgical clip applier when preparing or using.

## Claims

1. A surgical clip applier, **characterized in that** the surgical clip applier comprises an outer tube (10) with a jaw (14) protruding from a distal end of the outer tube (10) and a handle (20) mounted at a proximal end of the outer tube (10), in the outer tube (10) from top to bottom sequentially arranged comprising:
a shifting plate (11) with a proximal end connected to the handle (20) presenting a reciprocate movement relative to the outer tube (10) from a movement of the handle (20), multiple shifting elastic leaves (111) arranged apart to each other in a predetermined distance (D) on the shifting plate (11) along an axis, each shifting elastic leaf (111) protruding toward the distal end of the outer tube (10) downwardly, and a feeding portion (15) mounted at a distal end of the shifting plate (11) for moving the distal most clip (A) into the jaw; and
a fixing plate (12), multiple fixing elastic leaves (121) arranged apart to each other on the fixing plate (12) corresponding to the shifting elastic leaves (111) in position, each fixing elastic leaf (121) protruding toward the distal end of the outer tube (10) upwardly, a storage space (13) defined at the space between the shifting plate (11) and the fixing plate (12), the storage space (13) holding multiple clips (A) in sequence inside, a clip pusher (16) moved along the storage space (13) by the movement of shifting plate (11) and pushing the proximal most clip (A) toward to the distal end of outer tube (10), wherein
after the last clip (A) is ejected, the clip pusher (16) is moved to a distal most position, when the handle (20) is triggered again, the feeding portion (15) pushes the clip pusher (16) into the jaw (14).

2. The surgical clip applier as claimed in claim 1, wherein the feeding portion (15) protrudes distally at a middle side of the distal end of shifting plate (11); and
an abutting portion (162) is formed at a proximal side of the clip pusher (16) with a position corresponding to the feeding portion (15), when the clip pusher (16) is moved to the distal most position, the feeding portion (15) abuts the proximal side of the abutting portion (162).

3. The surgical clip applier as claimed in claim 2, wherein the multiple shifting elastic leaves (111) protrude toward the distal end downwardly from one side of the shifting plate (11); and
the proximal side of the clip pusher (16) has a pushing portion (161) which is located at a position corresponding to the multiple shifting elastic leaves (111), and the multiple shifting elastic leaves (111) push the pushing portion (161) when moving distally.

4. The surgical clip applier as claimed in claim 3, wherein the shifting elastic leaves (111) are detachably combined with the pushing portion (161).

5. The surgical clip applier as claimed in claim 4, wherein the pushing portion (161) is a notch defined in the proximal side surface of the clip pusher (16), and the shifting elastic leaves (111) is fixed by being inserted into the notch when moving distally.

6. The surgical clip applier as claimed in claim 3, wherein the abutting portion (162) is located at the distal side of the pushing portion (161).

7. The surgical clip applier as claimed in claim 6, wherein the jaw (14) has two jaw units (141) which are mounted apart from each other, the two jaw units (141) are narrowed or expanded according to the movement driven by handle (20), one side of the two jaw units (141) corresponding to each other can be defined as an inner wall, the inner wall of each jaw unit (141) is provided with a feeding notch (142), and the clips (A) or the clip pusher (16) can follow the feeding notches (142) to move into the jaw (14)

8. The surgical clip applier as claimed in claim 7, wherein the jaw (14) extends a stop portion (143) radially in order to axially stop the clips (A) or the clip pusher (16) falling off from the two jaw units (141).

9. The surgical clip applier as claimed in claim 1 to 8, wherein the handle (20) is a trigger structure and comprises:
a fixing handle; and
a pressing handle (21), a pivot portion (211not found in the drawings??) formed at a top side of the pressing handle (21),wherein when the pressing handle (21) is pivoted toward the fixing handle, the pressing handle (21) drives the shifting plate (11) to the distal end of the outer tube (10).

10. The surgical clip applier as claimed in claim 9, wherein:
a tooth-locking structure (30) is located near the pressing handle (21) and comprises:
a ratchet (31) protruding from the pivot portion (211);
a toothed block (32) formed with a toothed edge (321) facing the ratchet (31), when the handle (20) is triggered or released, the toothed edge (321) engages and moves along the ratchet (31) unidirectionally according to a pivot direction of the pressing handle (21); and
a connect springs (322) settled at one side of the ratchet (31) corresponding to the toothed edge (321) of the toothed block (32).
